# EUROPÄISCHE PATENTSCHRIFT

(11) **EP 2 858 622 B1**
(45) Veröffentlichungstag und Bekanntmachung des Hinweises auf die Patenterteilung: **31.08.2016**
(21) Anmeldenummer: 13728690.2
(22) Anmeldetag: 29.05.2013
(51) Int. Cl.: A61K 8/46, A61K 8/44, A61Q 5/02, A61Q 19/10, A61K 8/42

(54) **VERWENDUNG VON N-METHYL-N-ACYLGLUCAMINEN ALS VERDICKER IN TENSIDLÖSUNGEN**
USE OF N-METHYL-N-ACYLGLUCAMINES AS THICKENING AGENTS IN SURFACTANT SOLUTIONS
UTILISATION DE N-MÉTHYL-N-ACYLGLUCAMINES COMME ÉPAISSISSANTS DANS DES SOLUTIONS TENSIOACTIVES

(30) Priorität: 30.05.2012 DE 102012010652; 06.05.2013 DE 102013208258
(43) Veröffentlichungstag der Anmeldung: 15.04.2015
(73) Patentinhaber: Clariant International Ltd., 4132 Muttenz (CH)
(72) Erfinder: KLUG, Peter, 63762 Großostheim (DE); MILDNER, Carina, 65931 Frankfurt am Main (DE)
(74) Vertreter: Holmes, Rosalind
(86) Internationale Anmeldenummer: PCT/EP2013/061100
(87) Internationale Veröffentlichungsnummer: WO 2013/178697

(56) Entgegenhaltungen:
- WO-A1-92/06162
- WO-A1-93/18125
- WO-A1-94/12608
- WO-A1-95/17880
- WO-A1-96/10386
- US-A- 5 716 922

## Beschreibung

Die Erfindung betrifft die Verwendung von N-Methyl-N-acylglucaminen als Verdicker in Tensidlösungen sowie kosmetische Zusammensetzungen, enthaltend solche Tensidlösungen.

An kosmetische Produkte werden hohe Anforderungen gestellt. Sie sollen ein klares Erscheinungsbild zeigen, toxikologisch und ökotoxikologisch unbedenklich sein, ein angenehmes Hautgefühl erzeugen und ein ausgezeichnetes rheologisches Verhalten haben, das über einen breiten pH-Bereich konstant ist.

Wasser- oder lösungsmittelhaltige Mehrkomponentensysteme wie Emulsionen oder Suspensionen werden häufig aus ökonomischen Gründen, aus anwendungstechnischen Gründen oder aus Stabilitätsgründen auf höhere Viskositäten eingestellt bzw. verdickt.

So kann z.B. durch Erhöhung der Viskosität der externen oder internen Phase von Emulsionen oder Suspensionen erreicht werden, dass die Zeit bis zur Entmischung der Komponenten eines solchen Systems deutlich verlängert werden kann, was sich in einer Verlängerung der Lagerzeit bemerkbar macht. Durch Erhöhung der Viskosität wird auch bei vielen Produkten deren gleichmäßige Verteilbarkeit insbesondere auf unebenen Flächen verbessert.

Durch die gleichmäßigere Verteilung und verlängerte Einwirkdauer wird so die Wirksamkeit erhöht. Neben den erwähnten anwendungstechnischen Vorteilen bietet die hohe Viskosität solcher Präparate auch weitere Vorteile bei der Herstellung, Verpackung, Abfüllung und Lagerung sowie beim Transport.

In der Fachliteratur wird eine Vielzahl von unterschiedlichen Systemen angegeben, um die rheologischen Eigenschaften von wässrigen oder lösungsmittelhaltigen Systemen, Emulsionen oder Suspensionen einzustellen. Bekannt sind beispielsweise Celluloseether und andere Cellulosederivate (z.B. Carboxymethylcellulose, Hydroxyethylcellulose), Gelatine, Stärke und Stärkederivate, Natriumalginate, Fettsäurepolyethylenglykolester, Agar-Agar, Traganth oder Dextrine. Als synthetische Polymere kommen verschiedene Materialien zum Einsatz, wie z.B. Polyvinylalkohole, Polyacrylamide, Polyacrylsäure und verschiedene Salze der Polyacrylsäure, Polyvinylpyrrolidon, Polyvinylmethylether, Polyethylenoxide, Copolymere aus Maleinsäureanhydrid und Vinylmethylether, sowie diverse Mischungen und Copolymerisate aus den oben angegebenen Verbindungen.

Die genannten Verbindungen zeigen jedoch bei der Anwendung vielfältige Nachteile. So sind z.B. die Cellulosederivate bzw. allgemein die auf natürlichen Rohstoffen basierenden Materialien und die daraus resultierenden Formulierungen sehr anfällig gegen Bakterien. Anwendungstechnisch fallen sie zumeist durch die Bildung unangenehmer, "faden- ziehender" Gele auf. Fettsäurepolyethylenglykolester neigen in Gegenwart von Wasser zur Hydrolyse, die dabei entstehenden unlöslichen Fettsäuren verursachen unerwünschte Trübungen. Verdickungsmittel natürlichen Ursprungs (z.B. Agar-Agar oder Traganth) weisen je nach Herkunft eine stark schwankende Zusammensetzung auf.

In der EP-A 0 285 768 werden N- Polyhydroxyalkylfettsäureamide als Verdickungsmittel für flüssige wässrige Tensidsysteme vorgeschlagen. In den Anwendungsbeispielen dieser Druckschrift wird N-Methylkokosfettsäureglucamid als Verdicker in einem Ethersulfat-Paraffinsulfonat-Mischsystem eingesetzt.

Obwohl mit einem solchen System bereits gute Ergebnisse erzielt werden, bleibt doch Raum für Verbesserungen, beispielsweise was die Verdickerleistung angeht. Aufgabe war es daher, Verdicker für Tensidsysteme bereitzustellen, die gegenüber den bekannten Systemen eine höhere Verdickerleistung zeigen.

Es wurde gefunden, dass bestimmte N-Methyl-N-acylglucamine sich in besonderer Weise als Verdicker für Tensidsysteme eignen, die Alkylethersulfate oder Alkylsulfate, insbesondere in Kombination mit Betainen, enthalten.

Gegenstand der Erfindung ist daher die Verwendung von N-Methyl-N-acylglucaminen als Verdicker in einem wässrigen Tensidsystem, enthaltend mindestens ein Alkylethersulfat und/oder mindestens ein Alkylsulfat, wobei mindestens 60 Gew.-% der genannten N-Methyl-N-acylglucamine eine C₁₂, C₁₄- oder ungesättigte C₁₈-Acylgruppe aufweisen, der Gehalt an N-Methyl-N-acylglucaminen mit einer Acylgruppe kürzer als C₁₂ geringer als 5 % ist und der Anteil an N-Methyl-N-acylglucaminen, die eine ungesättigte C₁₈-Acylgruppe enthalten, bei mindestens 8 Gew.-% liegt.

Erfindungsgemäß eingesetzte N-Methyl-N-acylglucamine mit C₁₂- und C₁₄-Acylgruppen und Fettsäureethersulfaten oder Fettsäuresulfaten sind aus der WO 92/06158 beziehungsweise WO 92/06162 zur Verwendung in Spül- und Reinigungsmitteln bekannt. Eine besondere Eignung der erfindungsgemäßen Glucamid/Tensid-Kombinationen als Verdicker insbesondere für kosmetische Zusammensetzungen, lässt sich daraus jedoch nicht ableiten.

Die erfindungsgemäß verwendeten N-Methyl-N-acylglucamine, auch als N-Methyl-N-1-Desoxisorbityl-Fettsäureamide bekannt, enthalten mindestens 60 Gew.-% N-Methyl-N-acylglucamine, die eine C₁₂-, C₁₄- oder eine ungesättigten C₁₈-Acylgruppe enthalten (im Folgenden "C_{12/18}-N-Methyl-N-acylglucamine" genannt) und gleichzeitig weniger als 5 Gew-% N-Methyl-N-acylglucamine, die einen Fettsäurerest < C₁₂ enthalten. Der Anteil an N-Methyl-N-acylglucaminen, die eine ungesättigte C₁₈-Acylgruppe enthalten, liegt bei mindestens 8 Gew.-%. Die folgenden Gew.-%-Angaben beziehen sich auf die Gesamtmenge an N-Methyl-N-acylglucaminen (100 Gew.-%).

Insbesondere bevorzugt als Komponente a) sind gesättigte N-Methyl-N-acylglucamine der Formel (I), wobei der Acylrest R^{a}CO abgeleitet ist von der Laurinsäure, der Myristinsäure, der Ölsäure, der Linolsäure oder der Linolensäure:

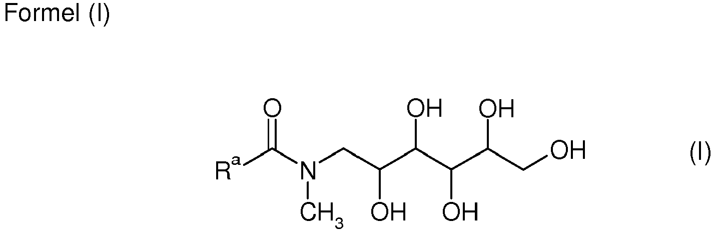

Der Anteil an N-Methyl-N-acylglucaminen, die eine C₁₂-C₁₄- oder eine ungesättigte C₁₈-Acylgruppe enthalten, liegt bei mindestens 60, bevorzugt 70, besonders bevorzugt 80 und ganz besonders bevorzugt 90, Gew.-%.

Daneben enthalten die erfindungsgemäß als Verdicker verwendeten N-Methyl-N-acylglucamine geringe Anteile an von kurzkettigen und/oder langkettigen Fettsäuren abgeleiteten N-Methyl-N-acylglucaminen, insbesondere solche, welche C₁-C₄-Acyl, C₆-, C₈-, C₁₀-, C₁₆-, C₁₈- und/oder C₂₀-Acylgruppen enthalten.

Der Anteil der N-Methyl-N-acylglucamine, die eine Acylgruppe < C₁₂ enthalten liegt bei weniger als 5, bevorzugt 3, besonders bevorzugt 2, Gew.-%.

Der Anteil an N-Methyl-N-acylglucaminen, die eine ungesättigte C₁₈-Acylgruppe enthalten, liegt bei mindestens 8, bevorzugt mindestens 15, Gew.-%.

Besonders bevorzugt liegt der Anteil an N-Methyl-N-acylglucaminen, die eine C₁₂-, C₁₄- oder einen ungesättigte C₁₈-Acylgruppe enthalten, bei mindestens 70 Gew.-% und der Anteil an N-Methyl-N-acylglucaminen, die eine Acylgruppe < C₁₂ enthalten bei weniger als 3 %.

Insbesondere bevorzugt liegt der Anteil an N-Methyl-N-acylglucaminen, die eine C₁₂-, C₁₄- oder eine ungesättigten C₁₈-Acylgruppe enthalten, bei mindestens 80 Gew.-% und der Anteil an N-Methyl-N-acylglucaminen, die eine Acylgruppe < C₁₂ enthalten bei weniger als 2 %.

In einer weiteren Ausführungsform liegt der Anteil an N-Methyl-N-acylglucaminen, die eine C₁₂- oder eine C₁₄-Acylgruppe enthalten, bei mindestens 90 Gew.-% und der Anteil an N-Methyl-N-acylglucaminen, die eine Acylgruppe < C₁₂ enthalten bei weniger als 2 %.

In einer weiteren bevorzugten Ausführungsform liegt der Anteil an N-Methyl-N-acylglucaminen, die eine C₁₂-, C₁₄- oder eine ungesättigten C₁₈-Acylgruppe enthalten, bei mindestens 60 Gew.-% und der Anteil an N-Methyl-N-acylglucaminen, die eine Acylgruppe < C₁₂ enthalten bei weniger als 5 %, wobei der Anteil der Anteil an N-Methyl-N-acylglucaminen, die eine ungesättigt C₁₈-Acylgruppe enthalten, bei mindestens 15 Gew.-% liegt.

In einer weiteren bevorzugten Ausführungsform liegt der Anteil an N-Methyl-N-acylglucaminen, die eine C₁₂-, C₁₄- oder eine ungesättigten C₁₈-Acylgruppe enthalten, bei mindestens 60 Gew.-% und der Anteil an N-Methyl-N-acylglucaminen, die eine Acylgruppe < C₁₂ enthalten bei weniger als 3 %, wobei der Anteil der Anteil an N-Methyl-N-acylglucaminen, die eine ungesättigt C₁₈-Acylgruppe enthalten, bei mindestens 8 Gew.-% liegt.

In einer weiteren bevorzugten Ausführungsform liegt der Anteil an N-Methyl-N-acylglucaminen, die eine C₁₂-, C₁₄- oder eine ungesättigten C₁₈-Acylgruppe enthalten, bei mindestens 60 Gew.-% und der Anteil an N-Methyl-N-acylglucaminen, die eine Acylgruppe < C₁₂ enthalten bei weniger als 3 %, wobei der Anteil der Anteil an N-Methyl-N-acylglucaminen, die eine ungesättigt C₁₈-Acylgruppe enthalten, bei mindestens 15 Gew.-% liegt.

In einer weiteren bevorzugten Ausführungsform liegt der Anteil an N-Methyl-N-acylglucaminen, die eine C₁₂-, C₁₄- oder eine ungesättigten C₁₈-Acylgruppe enthalten, bei mindestens 60 Gew.-% und der Anteil an N-Methyl-N-acylglucaminen, die eine Acylgruppe < C₁₂ enthalten bei weniger als 2 %, wobei der Anteil der Anteil an N-Methyl-N-acylglucaminen, die eine ungesättigt C₁₈-Acylgruppe enthalten, bei mindestens 8 Gew.-% liegt.

In einer weiteren bevorzugten Ausführungsform liegt der Anteil an N-Methyl-N-acylglucaminen, die eine C₁₂-, C₁₄- oder eine ungesättigten C₁₈-Acylgruppe enthalten, bei mindestens 60 Gew.-% und der Anteil an N-Methyl-N-acylglucaminen, die eine Acylgruppe < C₁₂ enthalten bei weniger als 2 %, wobei der Anteil der Anteil an N-Methyl-N-acylglucaminen, die eine ungesättigt C₁₈-Acylgruppe enthalten, bei mindestens 15 Gew.-% liegt.

In einer weiteren bevorzugten Ausführungsform liegt der Anteil an N-Methyl-N-acylglucaminen, die eine C₁₂-, C₁₄- oder eine ungesättigten C₁₈-Acylgruppe enthalten, bei mindestens 70 Gew.-% und der Anteil an N-Methyl-N-acylglucaminen, die eine Acylgruppe < C₁₂ enthalten bei weniger als 5 %, wobei der Anteil der Anteil an N-Methyl-N-acylglucaminen, die eine ungesättigt C₁₈-Acylgruppe enthalten, bei mindestens 8 Gew.-% liegt.

In einer weiteren bevorzugten Ausführungsform liegt der Anteil an N-Methyl-N-acylglucaminen, die eine C₁₂-, C₁₄- oder eine ungesättigten C₁₈-Acylgruppe enthalten, bei mindestens 70 Gew.-% und der Anteil an N-Methyl-N-acylglucaminen, die eine Acylgruppe < C₁₂ enthalten bei weniger als 5 %, wobei der Anteil der Anteil an N-Methyl-N-acylglucaminen, die eine ungesättigt C₁₈-Acylgruppe enthalten, bei mindestens 15 Gew.-% liegt.

In einer weiteren bevorzugten Ausführungsform liegt der Anteil an N-Methyl-N-acylglucaminen, die eine C₁₂-, C₁₄- oder eine ungesättigten C₁₈-Acylgruppe enthalten, bei mindestens 70 Gew.-% und der Anteil an N-Methyl-N-acylglucaminen, die eine Acylgruppe < C₁₂ enthalten bei weniger als 3 %, wobei der Anteil der Anteil an N-Methyl-N-acylglucaminen, die eine ungesättigt C₁₈-Acylgruppe enthalten, bei mindestens 8 Gew.-% liegt.

In einer weiteren bevorzugten Ausführungsform liegt der Anteil an N-Methyl-N-acylglucaminen, die eine C₁₂-, C₁₄- oder eine ungesättigten C₁₈-Acylgruppe enthalten, bei mindestens 70 Gew.-% und der Anteil an N-Methyl-N-acylglucaminen, die eine Acylgruppe < C₁₂ enthalten bei weniger als 3 %, wobei der Anteil der Anteil an N-Methyl-N-acylglucaminen, die eine ungesättigt C₁₈-Acylgruppe enthalten, bei mindestens 15 Gew.-% liegt.

In einer weiteren bevorzugten Ausführungsform liegt der Anteil an N-Methyl-N-acylglucaminen, die eine C₁₂-, C₁₄- oder eine ungesättigten C₁₈-Acylgruppe enthalten, bei mindestens 70 Gew.-% und der Anteil an N-Methyl-N-acylglucaminen, die eine Acylgruppe < C₁₂ enthalten bei weniger als 2 %, wobei der Anteil der Anteil an N-Methyl-N-acylglucaminen, die eine ungesättigt C₁₈-Acylgruppe enthalten, bei mindestens 8 Gew.-% liegt.

In einer weiteren bevorzugten Ausführungsform liegt der Anteil an N-Methyl-N-acylglucaminen, die eine C₁₂-, C₁₄- oder eine ungesättigten C₁₈-Acylgruppe enthalten, bei mindestens 70 Gew.-% und der Anteil an N-Methyl-N-acylglucaminen, die eine Acylgruppe < C₁₂ enthalten bei weniger als 2 %, wobei der Anteil der Anteil an N-Methyl-N-acylglucaminen, die eine ungesättigt C₁₈-Acylgruppe enthalten, bei mindestens 15 Gew.-% liegt.

In einer weiteren bevorzugten Ausführungsform liegt der Anteil an N-Methyl-N-acylglucaminen, die eine C₁₂-, C₁₄- oder eine ungesättigten C₁₈-Acylgruppe enthalten, bei mindestens 80 Gew.-% und der Anteil an N-Methyl-N-acylglucaminen, die eine Acylgruppe < C₁₂ enthalten bei weniger als 5 %, wobei der Anteil der Anteil an N-Methyl-N-acylglucaminen, die eine ungesättigt C₁₈-Acylgruppe enthalten, bei mindestens 8 Gew.-% liegt.

In einer weiteren bevorzugten Ausführungsform liegt der Anteil an N-Methyl-N-acylglucaminen, die eine C₁₂-, C₁₄- oder eine ungesättigten C₁₈-Acylgruppe enthalten, bei mindestens 80 Gew.-% und der Anteil an N-Methyl-N-acylglucaminen, die eine Acylgruppe < C₁₂ enthalten bei weniger als 5 %, wobei der Anteil der Anteil an N-Methyl-N-acylglucaminen, die eine ungesättigt C₁₈-Acylgruppe enthalten, bei mindestens 15 Gew.-% liegt.

In einer weiteren bevorzugten Ausführungsform liegt der Anteil an N-Methyl-N-acylglucaminen, die eine C₁₂-, C₁₄- oder eine ungesättigten C₁₈-Acylgruppe enthalten, bei mindestens 80 Gew.-% und der Anteil an N-Methyl-N-acylglucaminen, die eine Acylgruppe < C₁₂ enthalten bei weniger als 3 %, wobei der Anteil der Anteil an N-Methyl-N-acylglucaminen, die eine ungesättigt C₁₈-Acylgruppe enthalten, bei mindestens 8 Gew.-% liegt.

In einer weiteren bevorzugten Ausführungsform liegt der Anteil an N-Methyl-N-acylglucaminen, die eine C₁₂-, C₁₄- oder eine ungesättigten C₁₈-Acylgruppe enthalten, bei mindestens 80 Gew.-% und der Anteil an N-Methyl-N-acylglucaminen, die eine Acylgruppe < C₁₂ enthalten bei weniger als 3 %, wobei der Anteil der Anteil an N-Methyl-N-acylglucaminen, die eine ungesättigt C₁₈-Acylgruppe enthalten, bei mindestens 15 Gew.-% liegt.

In einer weiteren bevorzugten Ausführungsform liegt der Anteil an N-Methyl-N-acylglucaminen, die eine C₁₂-, C₁₄- oder eine ungesättigten C₁₈-Acylgruppe enthalten, bei mindestens 80 Gew.-% und der Anteil an N-Methyl-N-acylglucaminen, die eine Acylgruppe < C₁₂ enthalten bei weniger als 2 %, wobei der Anteil der Anteil an N-Methyl-N-acylglucaminen, die eine ungesättigt C₁₈-Acylgruppe enthalten, bei mindestens 8 Gew.-% liegt.

In einer weiteren bevorzugten Ausführungsform liegt der Anteil an N-Methyl-N-acylglucaminen, die eine C₁₂-, C₁₄- oder eine ungesättigten C₁₈-Acylgruppe enthalten, bei mindestens 80 Gew.-% und der Anteil an N-Methyl-N-acylglucaminen, die eine Acylgruppe < C₁₂ enthalten bei weniger als 2 %, wobei der Anteil der Anteil an N-Methyl-N-acylglucaminen, die eine ungesättigt C₁₈-Acylgruppe enthalten, bei mindestens 15 Gew.-% liegt.

In einer weiteren bevorzugten Ausführungsform liegt der Anteil an N-Methyl-N-acylglucaminen, die eine C₁₂-, C₁₄- oder eine ungesättigten C₁₈-Acylgruppe enthalten, bei mindestens 90 Gew.-% und der Anteil an N-Methyl-N-acylglucaminen, die eine Acylgruppe < C₁₂ enthalten bei weniger als 5 %, wobei der Anteil der Anteil an N-Methyl-N-acylglucaminen, die eine ungesättigt C₁₈-Acylgruppe enthalten, bei mindestens 8 Gew.-% liegt.

In einer weiteren bevorzugten Ausführungsform liegt der Anteil an N-Methyl-N-acylglucaminen, die eine C₁₂-, C₁₄- oder eine ungesättigten C₁₈-Acylgruppe enthalten, bei mindestens 90 Gew.-% und der Anteil an N-Methyl-N-acylglucaminen, die eine Acylgruppe < C₁₂ enthalten bei weniger als 5 %, wobei der Anteil der Anteil an N-Methyl-N-acylglucaminen, die eine ungesättigt C₁₈-Acylgruppe enthalten, bei mindestens 15 Gew.-% liegt.

In einer weiteren bevorzugten Ausführungsform liegt der Anteil an N-Methyl-N-acylglucaminen, die eine C₁₂-, C₁₄- oder eine ungesättigten C₁₈-Acylgruppe enthalten, bei mindestens 90 Gew.-% und der Anteil an N-Methyl-N-acylglucaminen, die eine Acylgruppe < C₁₂ enthalten bei weniger als 3 %, wobei der Anteil der Anteil an N-Methyl-N-acylglucaminen, die eine ungesättigt C₁₈-Acylgruppe enthalten, bei mindestens 8 Gew.-% liegt.

In einer weiteren bevorzugten Ausführungsform liegt der Anteil an N-Methyl-N-acylglucaminen, die eine C₁₂-, C₁₄- oder eine ungesättigten C₁₈-Acylgruppe enthalten, bei mindestens 90 Gew.-% und der Anteil an N-Methyl-N-acylglucaminen, die eine Acylgruppe < C₁₂ enthalten bei weniger als 3 %, wobei der Anteil der Anteil an N-Methyl-N-acylglucaminen, die eine ungesättigt C₁₈-Acylgruppe enthalten, bei mindestens 15 Gew.-% liegt.

In einer weiteren bevorzugten Ausführungsform liegt der Anteil an N-Methyl-N-acylglucaminen, die eine C₁₂-, C₁₄- oder eine ungesättigten C₁₈-Acylgruppe enthalten, bei mindestens 90 Gew.-% und der Anteil an N-Methyl-N-acylglucaminen, die eine Acylgruppe < C₁₂ enthalten bei weniger als 2 %, wobei der Anteil der Anteil an N-Methyl-N-acylglucaminen, die eine ungesättigt C₁₈-Acylgruppe enthalten, bei mindestens 8 Gew.-% liegt.

In einer weiteren bevorzugten Ausführungsform liegt der Anteil an N-Methyl-N-acylglucaminen, die eine C₁₂-, C₁₄- oder eine ungesättigten C₁₈-Acylgruppe enthalten, bei mindestens 90 Gew.-% und der Anteil an N-Methyl-N-acylglucaminen, die eine Acylgruppe < C₁₂ enthalten bei weniger als 2 %, wobei der Anteil der Anteil an N-Methyl-N-acylglucaminen, die eine ungesättigt C₁₈-Acylgruppe enthalten, bei mindestens 15 Gew.-% liegt.

Die N-Methyl-N-acylglucamine können, wie in EP-A 0 550 637 B1 und EP-A 0 285 768 beschrieben, durch Umsetzung der entsprechenden Fettsäureester bzw. Fettsäureestergemische mit N-Methylglucamin in Gegenwart eines Hydroxylgruppen oder Alkoxylgruppen aufweisenden Lösungsmittels hergestellt werden. Geeignete Lösungsmittel sind beispielsweise C₁-C₄-Monoalkohole, Ethylenglykol, Propylenglykol, Glycerin sowie alkoxylierte Alkohole. Bevorzugt ist 1,2-Propylenglykol. N-Methylglucamin kann, wie ebenfalls in EP 0 550 637 A1 beschrieben, durch reduktive Aminierung von Glukose mit Methylamin erhalten werden. Geeignete Fettsäureester, die mit den N-Methylglucaminen zu erfindungsgemäßen Glucamiden umgesetzt werden, sind im Allgemeinen die Methylester, die durch Umesterung aus natürlichen Fetten und Ölen, beispielsweise den Triglyceride, gewonnen werden.

Als ungesättigte C₁₈-Acylgruppen im Sinne der Erfindung sind Fettsäurereste mit einer oder mehreren Doppelbindungen zu verstehen. Bevorzugt sind hierbei Reste, die sich von der Ölsäure, der Linol- und der Linolensäure ableiten.

Die wässrigen Tensidlösungen enthalten ein oder mehrere anionische Tenside aus der Gruppe der Alkylsulfate und Alkylethersulfate.

Bevorzugte Alkylsulfate sind die C₈-C₂₀-Alkylsulfate, insbesondere die linearen C₈-C₂₀-Alkylsulfate in Form ihrer Natrium-, Kalium- oder Ammoniumsalze. Beispiele für Alkylsulfate sind Laurylsulfat, Cocosalkylsulfat und Talgalkylsulfat. Besonders bevorzugt ist Laurylsulfat.

Bevorzugte Alkylethersulfate sind die C₈C₂₀-Alkylethersulfate, besonders bevorzugt sind die linearen C₈C₂₀-Alkylethersulfate, insbesondere die von den ethoxylierten Fettalkoholen abgeleiteten Alkylglykolethersulfate, in Form ihrer Natrium-, Kalium- oder Ammoniumsalze. Beispiele für Alkylethersulfate sind Laurylethersulfat, Cocosalkylethersulfat und Talgalkylethersulfat. Beispiele für Glykolethersulfate sind Lauryltriethylenglykolethersulfat, Cocosalkyltriethylenglykolethersulfat und Talgalkylhexaethylenglykolethersulfat. Insbesondere bevorzugt ist Laurylglykolethersulfat, beispielsweise, Lauryldiethylenglykolethersulfat oder Lauryltriethylenglykolethersulfat, speziell in Form der Natriumsalze.

Vorzugsweise enthalten die wässrigen Tensidlösungen neben dem mindestens einen anionischen Tensid ein Betain-Tensid.

Betain-Tenside enthalten im selben Molekül eine kationische Gruppe, insbesondere eine Ammonium-Gruppe, und eine anionische Gruppe, die eine Carboxylat-Gruppe, Sulfat-Gruppe oder Sulfonat-Gruppe sein kann. Geeignete Betaine sind Alkylbetaine wie Cocobetain oder Fettsäurealkylamidopropylbetaine, beispielsweise Cocosacylamidopropyldimethylbetain, C₁₂-C₁₈-Dimethylaminohexanoate oder C₁₀-C₁₈-Acylamidopropandimethylbetaine.

In einer bevorzugten Ausführungsform der Erfindung enthalten die wässrigen Tensidlösungen ein oder mehrere Amidopropylbetaine der allgemeinen Formel (II), 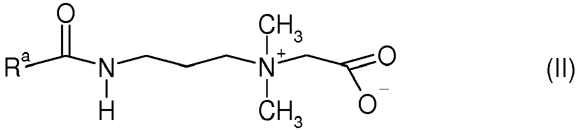 worin R^{a} eine lineare oder verzweigte gesättigte C₇-C₂₁ Alkylgruppe oder eine lineare oder verzweigte ein- oder mehrfach ungesättigte C₇-C₂₁ Alkenylgruppe ist.

In einer weiteren bevorzugten Ausführungsform der Erfindung enthalten die Tensidlösungen ein oder mehrere Betaine der Formel (III), 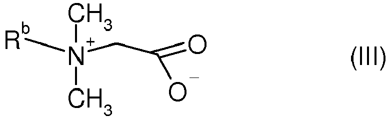 worin R^{b} eine lineare oder verzweigte gesättigte C₈-C₂₂ Alkylgruppe oder eine lineare oder verzweigte ein- oder mehrfach ungesättigte C₈-C₂₂ Alkenylgruppe ist.

In einer weiteren bevorzugten Ausführungsform der Erfindung enthalten die Tensidlösungen ein oder mehrere Sulfobetaine der Formel (IV), 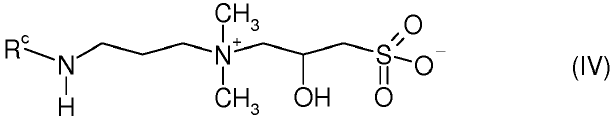 worin R^{c} eine lineare oder verzweigte gesättigte C₈-C₂₂ Alkylgruppe oder eine lineare oder verzweigte ein- oder mehrfach ungesättigte C₈-C₂₂ Alkenylgruppe ist.

Besonders bevorzugt enthalten die Tensidlösungen ein oder mehreren Betaintenside ausgewählt aus der Gruppe der Verbindungen bestehend aus den Amidopropylbetainen der Formel (II), den Betainen der Formel (III) und den Sulfobetainen der Formel (IV).

In einer insbesondere bevorzugten Ausführungsform der Erfindung enthalten die Tensidlösungen ein oder mehrere Betaintenside ausgewählt aus den Amidopropylbetainen der Formel (II).

In einer weiteren insbesondere bevorzugten Ausführungsform der Erfindung enthalten die Tensidlösungen ein oder mehrere Betaintenside ausgewählt aus den Betainen der Formel (III).

In einer weiteren insbesondere bevorzugten Ausführungsform der Erfindung enthalten die Tensidlösungen ein oder mehrere Betaintenside ausgewählt aus den Sulfobetainen der Formel (IV).

Vorzugsweise ist der Rest R^{a} in dem einen oder den mehreren Amidopropylbetainen der Formel (II) eine lineare oder verzweigte gesättigte C₇-C₁₇ Alkylgruppe. Unter den linearen und verzweigten gesättigten Alkylgruppen R^{a} sind die linearen gesättigten Alkylgruppen bevorzugt.

Besonders bevorzugt handelt es sich bei den Amidopropylbetainen der Formel (II) um Cocamidopropylbetaine.

Vorzugsweise ist der Rest R^{b} in dem einen oder den mehreren Betainen der Formel (III) eine lineare oder verzweigte gesättigte C₈-C₁₈-Alkylgruppe und besonders bevorzugt eine lineare oder verzweigte gesättigte C₁₂-C₁₈-Alkylgruppe. Unter den linearen und verzweigten gesättigten Alkylgruppen R^{b} sind die linearen gesättigten Alkylgruppen bevorzugt.

Vorzugsweise ist der Rest R^{c} in der einen oder den mehreren Sulfobetainen der Formel (IV) eine lineare oder verzweigte gesättigte C₈-C₁₈ Alkylgruppe und besonders bevorzugt eine lineare oder verzweigte gesättigte C₁₂-C₁₈ Alkylgruppe. Unter den linearen und verzweigten gesättigten Alkylgruppen R^{c} sind die linearen gesättigten Alkylgruppen bevorzugt.

Besonders bevorzugt enthalten die wässrigen Tensidlösungen Amidopropylbetaine der Formel (II) und/oder Alkylbetaine der Formel (III).

Weiterhin Gegenstand der Erfindung ist ein Verfahren zum Verdicken einer wässrigen Tensidlösung, enthaltend mindestens ein Alkylethersulfat und/oder mindestens ein Alkylsulfat, wobei der wässrigen Tensidlösung eine erfindungsgemäße N-Methyl-N-Acylglucamin-Mischung zugesesetzt wird. Bevorzugte Ausführungsformen sind die oben beschriebenen.

Die Erfindung wird durch die nachstehenden Beispiele näher erläutert.

### Beispiele

### Beispiele 2 bis 3 sowie Vergleichsbeispiele 1a und 1 bis 5

Die im Folgenden beschriebene Glucamide wurde nach EP 0 550 637 aus den korrespondierenden Fettsäuremethylestern und N-Methylglucamin in Gegenwart von 1,2 Propylenglykol als Lösemittel hergestellt und als Feststoff bestehend aus Aktivsubstanz und 1,2 Propylenglykol erhalten (alle Angaben in Gew.-%).

| Herstellbeispiel | Methylester | Triglycerid | Aktivsubstanz (%) | 1,2-Propylenglykol (%) | Schmelzpunkt (°C) |
|---|---|---|---|---|---|
| 1 | C12/14 (C12: 70 %, C14 30 %) | - | 90 | 10 | 85 |
| 2 | | Cocosöl (C8: 6 %; C 10: 6 %; C12:48% C14: 20 % C16: 10%; C18:2%, C18' = 8%) | 90 | 10 | 50 |
| 3 | C8/10 (C8: 60 %; C10: 40%) | - | 90 | 10 | 50 |
| 4 | C16/18 (C16: 60 %; C18: 40%) | - | 80 | 20 | 65 |
| 5 (erfindungsgemäß) | C12/18 ungesättigt (C12: 60 %, C14: 26 %, | - | 90 | 10 | 70 |
| | C16:4% C18: 1 % C18'(Öl-säure): 8 % C18" = 1% | | | | |
| 6 (erfindungsgemäß) | C16/18 ungesättigt C16:32% C18:8% C18' = 52% C18" = 8 % | - | 80 | 20 | 45 |
| 7 | Cocosmethylester C8: 6 %; C 10: 6 %; C12:48% C14: 20% C16: 10%; C18:2%, C18'= 8% | - | 90 | 10 | 55 |

Es wurden Tensidlösungen bestehend aus Natrium Laurylethersulfat [Ethoxylierungsgrad 2 EO] (Genapol LRO liq., Clariant), Cocamidopropylbetain (Genagen CAB 818, Clariant) und Zuckertenside gemäß der folgenden Tabelle hergestellt und durch Zugabe von Kochsalz auf eine einheitliche Viskosität von 5000 mPas angepasst. Der Gesamttensidgehalt betrug jeweils 12 %. Die zur Erzielung einer Viskosität von 5000 mPas benötigte Salzmenge wurde bestimmt.

| Beispiel | Verhältnis Natriumlaurylethersulfat : Cocamidopropylbetain : Zuckertensid | Zuckertensid | Benötigte Salzmenge für 5000 m Pas (%) |
|---|---|---|---|
| Vergleichsbeispiel 1a | 6:2:2 | Herstellbeispiel 1 | 1,0 |
| Beispiel 2 | 6:2:2 | Herstellbeispiel 5 | 0,75 |
| Beispiel 3 | 6:2:2 | Herstellbeispiel 6 | 0,80 |
| Vergleichsbeispiel 1 | 6:2:2 | Herstellbeispiel 2 | 1,70 |
| Vergleichsbeispiel 2 | 6:2:2 | Herstellbeispiel 3 | >3 |
| Vergleichsbeispiel 3 | 6:2:2 | Herstellbeispiel 4 | 1,40 |
| Vergleichsbeispiel 4 | 6:2:2 | Lauryl Glucoside (Plantacare 1200) | 1,75 |
| Vergleichsbeispiel 5 | 8:2:0 | Keines, Natrium Laurylethersulfat : | 2,5 |
| Vergleichsbeispiel 6 | 6:2:2 | Herstellbeispiel 7 | 1,3 |

Die erfindungsgemäßen Formulierungsbeispiele 2 und 3 benötigen gegenüber den nicht erfindungsgemäßen Vergleichsbeispielen bei weitem die niedrigste Salzkonzentrationen. Dies ist in Bezug auf die Haut- und Schleimhautverträglichkeit der kosmetischen Formulierungen vorteilhaft. Insbesondere ist an Beispiel 2 zu erkennen, dass ein Synergismus in der Verdickung auftritt, wenn man Glucamide mit Kettenlänge C12/14 und ungesättigten C18- Anteilen kombiniert. Dagegen besitzten die aus Kokosöl abgeleiteten und der EP 0 285 768 entsprechenden Produkte (Vergleichsbeispiele 1 und 6) keine den Beispielen 2-3 entsprechende Verdickungsleistung. Vergleichsbeispiel 3 zeigt, dass die Verdickungsleistung gesättigter C16/18 Glucamide nicht an die erfindungsgemäßen Zusammensetzungen heranreicht.

## Patentansprüche

1. Verwendung von N-Methyl-N-acylglucaminen als Verdicker in wässrigen Tensidlösungen, enthaltend ein oder mehrere anionische Tenside aus der Gruppe der Alkylethersulfate und Alkylsulfate, wobei die N-Methyl-N-acylglucamine mindestens 60 Gew.-% N-Methyl-N-acylglucamine mit einem C₁₂-, C₁₄- oder einen ungesättigten C₁₈- Fettsäurerest enthalten und gleichzeitig weniger als 5 Gew-% N-Methyl-N-acylglucamine, die einen Fettsäurerest < C₁₂ enthalten; wobei der Anteil an N-Methyl-N-acylglucaminen, die eine ungesättigte C₁₈-Acylgruppe enthalten, bei mindestens 8 Gew.-% liegt.

2. Verwendung nach Anspruch 1, wobei die mindestens 60 Gew.-% N-Methyl-N-acylglucamine Verbindungen der Formel (I) sind, 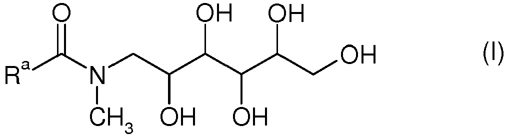 wobei der Rest R^{a} von Laurinsäure, Myristylsäure, Ölsäure, Linolsäure oder Linolensäure abgeleitet ist.

3. Verwendung nach Anspruch 1 oder 2, wobei der Anteil an N-Methyl-N-acylglucamiden, die eine C₁₂-, C₁₄- oder eine ungesättigte C₁₈-Acylgruppe enthalten, bei mindestens 70 Gew.-% liegt.

4. Verwendung nach Anspruch 3, wobei der Anteil an N-Methyl-N-acylglucamiden, die eine C₁₂-, C₁₄- oder eine ungesättigte C₁₈-Acylgruppe enthalten, bei mindestens 80 Gew.-% liegt.

5. Verwendung nach Anspruch 4, wobei der Anteil an N-Methyl-N-acylglucamiden, die eine C₁₂-, C₁₄- oder eine ungesättigte C₁₈-Acylgruppe enthalten, bei mindestens 90 Gew.-% liegt.

6. Verwendung nach einem der Ansprüche 1 bis 5, wobei der Anteil an N-Methyl-N-acylglucaminen, die eine Acylgruppe < C₁₂ enthalten, bei weniger als 3 Gew.-% liegt.

7. Verwendung nach Anspruch 6, wobei der Anteil an N-Methyl-N-acylglucaminen, die eine Acylgruppe < C₁₂ enthalten, bei weniger als 2 Gew.-% liegt.

8. Verwendung nach einem der Ansprüche 1 bis 7, wobei der Anteil an N-Methyl-N-acylglucaminen, die eine ungesättigte C₁₈-Acylgruppe enthalten, bei mindestens 15 Gew.-% liegt.

9. Verwendung nach einem der Ansprüche 1 bis 8, **dadurch gekennzeichnet, dass** die wässrigen Tensidlösungen ein Alkylsulfat und/oder ein Alkylethersulfat als anionisches Tensid und ein Betain-Tensid enthalten.

10. Verwendung nach einem der Ansprüche 1 bis 9, **dadurch gekennzeichnet, dass** die wässrigen Tensidlösungen ein lineares C₈-C₂₀-Alkylsulfat und/oder ein lineares C₈-C₂₀-Alkylethersulfat enthalten.

11. Verwendung nach Anspruch 10, **dadurch gekennzeichnet, dass** die wässrigen Tensidlösungen Laurylsulfat und/oder ein Laurylethersulfat enthalten.

12. Verwendung nach einem der Ansprüche 1 bis 11, **dadurch gekennzeichnet, dass** die wässrigen Tensidlösungen ein Acylamidopropylbetain oder ein Alkylbetain enthalten.

13. Verfahren zum Verdicken einer wässrigen Tensidlösung, enthaltend ein oder mehrere anionische Tenside aus der Gruppe der Alkylethersulfate und Alkylsulfate, wobei der wässrigen Tensidlösung N-Methyl-N-acyl-glucamine gemäß einem der Ansprüche 1 bis 8 zugesetzt werden.

## Claims

1. Use of N-methyl-N-acylglucamines as thickener in aqueous surfactant solutions, containing one or more anionic surfactants from the group of the alkyl ether sulfates and alkyl sulfates, wherein the N-methyl-N-acylglucamines contain at least 60 wt% of N-methyl-N-acylglucamines with a C₁₂-, C₁₄- or an unsaturated C₁₈-fatty acid residue and simultaneously less than 5 wt% of N-methyl-N-acylglucamines that contain a fatty acid residue < C₁₂; wherein the proportion of N-methyl-N-acylglucamines that contain an unsaturated C₁₈-acyl group is at least 8 wt%.

2. The use as claimed in claim 1, wherein the at least 60 wt% of N-methyl-N-acylglucamines are compounds of formula (I), 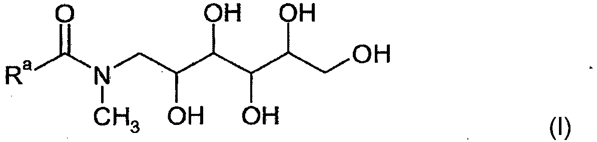 wherein the residue R^{a} is derived from lauric acid, myristyl acid, oleic acid, linoleic acid or linolenic acid.

3. The use as claimed in claim 1 or 2, wherein the proportion of N-methyl-N-acylglucamides that contain a C₁₂-, C₁₄- or an unsaturated C₁₈-acyl group is at least 70 wt%.

4. The use as claimed in claim 3, wherein the proportion of N-methyl-N-acylglucamides that contain a C₁₂-, C₁₄- or an unsaturated C₁₈-acyl group is at least 80 wt%.

5. The use as claimed in claim 4, wherein the proportion of N-methyl-N-acylglucamides that contain a C₁₂-, C₁₄- or an unsaturated C₁₈-acyl group is at least 90 wt%.

6. The use as claimed in one of claims 1 to 5, wherein the proportion of N-methyl-N-acylglucamines that contain an acyl group < C₁₂ is less than 3 wt%.

7. The use as claimed in claim 6, wherein the proportion of N-methyl-N-acylglucamines that contain an acyl group < C₁₂ is less than 2 wt%.

8. The use as claimed in one of claims 1 to 7, wherein the proportion of N-methyl-N-acylglucamines that contain an unsaturated C₁₈-acyl group is at least 15 wt%.

9. The use as claimed in one of claims 1 to 8, wherein the aqueous surfactant solutions contain an alkyl sulfate and/or an alkyl ether sulfate as anionic surfactant and a betaine surfactant.

10. The use as claimed in one of claims 1 to 9, wherein the aqueous surfactant solutions contain a linear C₈-C₂₀-alkyl sulfate and/or a linear C₈-C₂₀-alkyl ether sulfate.

11. The use as claimed in claim 10, wherein the aqueous surfactant solutions contain lauryl sulfate and/or a lauryl ether sulfate.

12. The use as claimed in one of claims 1 to 11, wherein the aqueous surfactant solutions contain an acylamidopropyl betaine or an alkyl betaine.

13. A method of thickening an aqueous surfactant solution containing one or more anionic surfactants from the group of the alkyl ether sulfates and alkyl sulfates, wherein N-methyl-N-acyl-glucamines as claimed in one of claims 1 to 8 are added to the aqueous surfactant solution.

## Revendications

1. Utilisation de N-méthyl-N-acylglucamines en tant qu'épaississants dans des solutions aqueuses de tensioactifs, contenant un ou plusieurs tensioactifs anioniques choisis dans le groupe des alkyléthersulfates et alkylsulfates, dans laquelle les N-méthyl-N-acylglucamines comprennent au moins 60 % en poids de N-méthyl-N-acylglucamines comportant un radical acide gras en C₁₂, C₁₄ ou un radical acide gras en C₁₈ insaturé, et en même temps moins de 5 % en poids de N-méthyl-N-acylglucamines qui comportent un radical acide gras en moins de C₁₂; la proportion de N-méthyl-N-acylglucamines qui comportent un groupe acyle en C₁₈ étant d'au moins 8 % en poids.

2. Utilisation selon la revendication 1, dans laquelle lesdits au moins 60 % en poids de N-méthyl-N-acylglucamines sont des composés de formule (I), 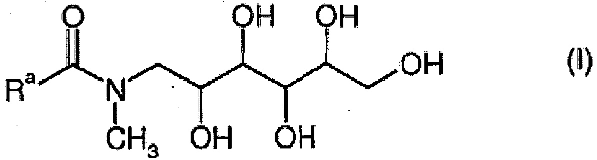 dans laquelle le radical R^{a} est dérivé de l'acide laurique, myristylique, oléique, linoléique ou linolénique.

3. Utilisation selon la revendication 1 ou 2, dans laquelle la proportion de N-méthyl-N-acylglucamines qui comportent un groupe acyle en C₁₂, C₁₄ ou un groupe acyle en C₁₈ insaturé est d'au moins 70 % en poids.

4. Utilisation selon la revendication 3, dans laquelle la proportion de **N**-méthyl-**N**-acylglucamines qui comportent un groupe acyle en C₁₂, C₁₄ ou un groupe acyle en C₁₈ insaturé est d'au moins 80 % en poids.

5. Utilisation selon la revendication 4, dans laquelle la proportion de N-méthyl-N-acylglucamines qui comportent un groupe acyle en C₁₂, C₁₄ ou un groupe acyle en C₁₈ insaturé est d'au moins 90 % en poids.

6. Utilisation selon l'une quelconque des revendications 1 à 5, dans laquelle la proportion de N-méthyl-N-acylglucamines qui comportent un groupe acyle en moins de C₁₂ est inférieure à 3 % en poids.

7. Utilisation selon la revendication 6, dans laquelle la proportion de N-méthyl-N-acylglucamines qui comportent un groupe acyle en moins de C₁₂ est inférieure à 2 % en poids.

8. Utilisation selon l'une quelconque des revendications 1 à 7, dans laquelle la proportion de N-méthyl-N-acylglucamines qui comportent un groupe acyle en C₁₈ insaturé est d'au moins 15 % en poids.

9. Utilisation selon l'une quelconque des revendications 1 à 8, **caractérisée en ce que** les solutions aqueuses de tensioactifs contiennent un alkylsulfate et/ou un alkyléthersulfate en tant que tensioactif anionique et un tensioactif de type bétaïne.

10. Utilisation selon l'une quelconque des revendications 1 à 9, **caractérisée en ce que** les solutions aqueuses de tensioactifs contiennent un alkyl(C₈-C₂₀)sulfate linéaire et/ou un alkyl(C₈-C₂₀)éthersulfate linéaire.

11. Utilisation selon la revendication 10, **caractérisée en ce que** les solutions aqueuses de tensioactifs contiennent un laurylsulfate et/ou un lauryléthersulfate.

12. Utilisation selon l'une quelconque des revendications 1 à 11, **caractérisée en ce que** les solutions aqueuses de tensioactifs contiennent une acylamidopropylbétaïne ou une alkylbétaïne.

13. Procédé pour l'épaississement d'une solution aqueuse de tensioactifs, contenant un ou plusieurs tensioactifs anioniques choisis dans le groupe des alkyléthersulfates et alkylsulfates, dans lequel on ajoute à la solution aqueuse de tensioactifs des N-méthyl-N-acylglucamines selon l'une quelconque des revendications 1 à 8.
